# EUROPEAN PATENT SPECIFICATION

(11) **EP 2 146 763 B1**
(45) Date of publication and mention of the grant of the patent: **14.09.2016**
(21) Application number: 08744538.3
(22) Date of filing: 28.03.2008
(51) Int. Cl.: A61M 11/00, A61M 15/00

(54) **METERED DOSE DISPENSING DEVICES**
SPENDERVORRICHTUNGEN FÜR ABGEMESSENE DOSEN
DISPOSITIFS DE DISTRIBUTION DE DOSE MESURÉE

(30) Priority: 22.05.2007 GB 0709684
(43) Date of publication of application: 27.01.2010
(73) Proprietor: 3M Innovative Properties Company, St. Paul, MN 55133-3427 (US)
(72) Inventor: BISHOP, Christopher, Bracknell Berkshire RG12 8HT (GB)
(74) Representative: Bergen, Katja
(86) International application number: PCT/US2008/058568
(87) International publication number: WO 2008/144107

(56) References cited:
- WO-A1-92/20391
- WO-A1-2007/054797
- WO-A2-2007/103712
- US-A- 3 994 421
- US-A- 5 392 768
- US-A- 5 988 496
- US-A1- 2003 178 020
- US-A1- 2004 139 964
- US-B2- 6 655 381
- US-B2- 6 953 039
- US-B2- 6 981 499

## Description

### BACKGROUND

The present invention relates to metered dose dispensing devices, in particular metered dose inhalers, comprising dose counter devices as well as dose counter devices of the type adapted to be used on such dispensing devices.

Metered medication dose dispensers, known as "inhalers", are commonly used for the treatment of asthma and other respiratory conditions. Metered dose dispensers typically include a medication dispensing canister and often an adaptor or housing for receiving the canister. The dispensing canister typically includes an aerosol container filled with a medicament that is formulated with a suitable propellant and equipped with a dispensing nozzle, fitted by means of a ferrule, such as a valve, in particular, a metered dose valve, comprising an elongate outlet member (e.g., a valve stem) movable between closed and discharge positions. The canister is generally not refillable, and is disposed of once the medication therein has been dispensed. The dispensing canister is typically used in conjunction with an adaptor that has a patient port (e.g., a mouthpiece or a port adapted for nasal use). The adaptor typically comprises a support block that has a socket adapted to receive the outlet member of the valve on the container, and has an orifice in communication with the socket and the patient port. The dispensing canister and the support block are reciprocally movable relative to each other along an axis to allow the outlet member to move to its discharge position during the operation or firing of the device, thereby dispensing a dose of the medicament from the container. The adaptor also typically includes an elongate portion extending opposite the support block and providing a chamber to house at least a portion of the container. There are many related design features of the adaptor and the dispensing canister that are employed in order to achieve the desired medicament dispensing performance (i.e., the dispensing of one metered amount or dose of sprayed medication of appropriate particle size distribution each time the dispenser is actuated by a user).

US 2003/178020 discloses an indicating device for indicating the number of metered dosages that have been dispensed from, or remain in, an aerosol container, and in particular, to an indicating device adapted to be mounted to the aerosol container.

US 2004/139964 discloses metered dose inhalers, and particularly it discloses an actuation inhibitor which precludes unintended or inadvertent actuation of the metered dose inhaler.

WO 92/20391 discloses a two-part aerosol inhalation device which maximizes the delivery of a medicament, such as an LH-RH analog or other pep tide, into the deepest part of the lung where it becomes most effective.

To dispense a dose of medication, a dispenser (inhaler) user normally squeezes or pushes down on the dispenser in an axial direction causing a relative movement of the canister into the adaptor towards the support block. It is useful for the user to know how many doses remain in his or her device (i.e., how much medicament by dosage is in the container of the dispensing canister). To this end, dose counter devices have been designed, some of which include a counter actuation button onto which the user squeezes or pushes down in the axial direction as he or she is actuating the dispenser to dispense a dose of medication. In this manner both the dose counter and the dispenser are actuated by the user. Such user-actuated dose counter devices are typically mounted onto the base of the dispensing canister (i.e. onto the closed end of the canister opposite to the end equipped with a dispensing nozzle) (top-mounted dose counters) or alternatively are provided on an external end of the adaptor at a position opposite to the closed end of the dispensing canister (bottom-mounted dose counters). In the following such dose counter devices will be referred to collectively as end-mounted dose counters.

### SUMMARY

It has been noted that end-mounted counters are prone to unintentional actuation, e.g. inter alia due to dropping the dispenser and hitting the counter actuation button, or setting the dispenser down on a table or similar surface "the wrong way" or too hard so that the counter actuation button is inadvertently actuated, leading to an incorrect count of the number of doses dispensed or remaining in the dispenser. This tendency towards unintentional counter actuation, with the resulting over-counting, is exacerbated by the fact that the force needed to actuate such end-mounted dose counters is typically arranged to be less than the force needed to actuate the dispenser (inhaler), in order to prevent any under-counting during routine use of the dispenser.

Surprisingly it has been found that by providing around the counter actuation button a crenelated parapet having two or more merlons, where the most axially outwardly facing surfaces of the merlons extend outwardly beyond a plane generally perpendicular to the axial direction and tangential to the most axially outwardly facing surface(s) of the top plate of the counter actuation button (which can be concave, convex or essentially flat in form) at the position of the counter actuation button at the point of actuation of the dose counter, it is possible to minimize the potential for unintentional actuation of the dose counter while still allowing the user good access to the counter actuation button to ensure proper and convenient operation during routine use of the dispenser.

Accordingly in one aspect of the present invention there is provided a medicinal dispensing device comprising a dose counter device for counting the doses of an aerosol medication dispensed from the dispensing device, said dispensing caused by an application of force in an axial direction on said dispensing device, the dose counter device comprising an actuation button and being operable by application of a force in the axial direction to the dispensing device, where the actuation button comprises a top plate and a side wall or side walls, as applicable, and wherein at the position of the counter actuation button at the point of actuation of the dose counter device there is a plane generally perpendicular to said axial direction and tangential to the most axially outwardly facing surface or surfaces of the top plate of the counter actuation button and wherein the dispensing device is provided with a crenelated parapet having two or more merlons, the merlons being positioned around the counter actuation button near the sidewall or sidewalls, as applicable, wherein the most axially outwardly facing surfaces of the merlons extend outwardly beyond said plane.

To further minimize or essentially eliminate the potential for unintentional actuation of the dose counter while still allowing the user good access to the counter actuation button, it is favorable to provide merlons where the most axially outwardly facing surfaces of the merlons are flush with or extend outwardly beyond a second plane generally perpendicular to the axial direction and tangential to the most axially outwardly facing surface(s) of the top plate of the counter actuation button at the position of the counter actuation button at rest.

Favorably the parapet is mounted on a component of the medicinal dispensing device or integral with a component of the medicinal dispensing device, said component not being the counter actuation button.

The dose counter device may be a top mounted dose counter device, e.g. where the dispensing device comprises a dispensing canister having an end equipped with a dispensing nozzle and a closed end, the dose counter device being mounted to the closed end of the dispensing canister.

Alternatively the dose counter device may be a bottom-mounted dose counter device, e.g. where the dispensing device comprises a dispensing canister having an end equipped with a dispensing nozzle and a closed end and a housing therefor, the dose counter device being mounted to or integral with an external end of the housing that is opposite to the closed end of the canister.

For dispensing devices including a top-mounted dose counter the parapet may be mounted on a dispensing canister of the dispensing device and/or a component of the dose counter device, or the parapet may be integral with a component of the dose counter device. Also for dispensing devices including a top-mounted dose counter device and a housing for the dispensing canister, the parapet may be mounted on or integral with the canister housing. For dispensing devices including a bottom-counted dose counter the parapet may be mounted onto the canister housing and/or a component of the dose counter, or the parapet may be integral with the canister housing and/or a component of the dose counter.

It will be appreciated by the skilled reader that in order to reduce or essentially eliminate the potential for unintended actuation of the dose counter the parapet is not to be mounted on or be integral with the counter actuation button itself.

Desirably the parapet is mounted on or is integral with a component of the dose counter, e.g. a counter housing or base.

Another aspect of the present invention is the provision of a dose counter device for use on a medicinal dispensing device and for counting the doses of an aerosol medication dispensed from the dispensing device, the dose counter device comprising an actuation button and being operable by application of a force in an axial direction on said actuation button, where the actuation button comprises a top plate and a side wall or side walls, as applicable, and wherein at the position of the actuation button at the point of actuation of the dose counter device there is a plane generally perpendicular to said axial direction and tangential to the most axially outwardly facing surface(s) of the top plate of the counter actuation button and wherein the dose counter device is provided with a crenelated parapet having two or more merlons, the merlons being positioned around the counter actuation button near the sidewall or sidewalls, as applicable, wherein the most axially outwardly facing surfaces of the merlons extend outwardly beyond said plane.

In more favorable embodiments of such dose counter devices, the merlons are provided such that the most axially outwardly facing surfaces of the merlons are flush with or extend outwardly beyond a second plane generally perpendicular to the axial direction and tangential to the most axially outwardly facing surface(s) of the top plate of the counter actuation button at the position of the counter actuation button at rest.

Such dose counter devices may be adapted to be mounted to the closed end of a dispensing canister of a dispensing device or adapted to be mounted to an external end of a canister housing that is opposite to the closed end of the canister.

A further example is the provision of a collar for use with a dose counter device comprising an actuation button and being operable by application of a force in an axial direction on said actuation button, where the actuation button comprises a top plate and a side wall or side walls, as applicable, or for use with a medicinal dispensing device comprising such a dose counter device, said collar being provided with a crenelated parapet having two or more merlons, the collar being configured and adapted to be mounted on a component of the dose counter device or a component of the medicinal dispensing device, respectively, said component not being the counter actuation button, such that the merlons will be positioned around the counter actuation button near the sidewall or sidewalls, as applicable, and wherein the most axially outwardly facing surfaces of the merlons extend outwardly beyond a plane generally perpendicular to said axial direction and tangential to the most axially outwardly facing surface(s) of the top plate of the counter actuation button at the position of the counter actuation button at the point of actuation of the dose counter device.

In more favorable examples of such collars, the collar is configured and adapted to be mounted on a component of the dose counter device or a component of the medicinal dispensing device, respectively, such that the most axially outwardly facing surfaces of the merlons will be flush with or extend outwardly beyond a second plane generally perpendicular to the axial direction and tangential to the most axially outwardly facing surface(s) of the top plate of the counter actuation button at the position of the counter actuation button at rest.

Dependent claims define further embodiments in accordance with the invention.

This summary is not intended to describe each disclosed embodiment or every implementation of the present invention. Many other novel advantages, features, and relationships will become apparent as this description proceeds. The figures and the description that follow more particularly exemplify illustrative embodiments.

### BRIEF DESCRIPTION OF THE DRAWINGS

The present invention will be further explained with reference to the attached figures, wherein like structure or system elements are referred to by like reference numerals throughout the several views.
FIGURE 1 is an isometric view of an exemplary dose counter device in accordance with the invention.
FIGURES 2a-c are cross-sections of the dose counter device shown in FIG. 1.
FIGURES 3a and 3b are exploded views of the components of the dose counter device of FIG. 1 and FIG. 2.
FIGURES 4a and 4b are partial cross-sections of an exemplary medicinal dispensing device in accordance with the invention, with the dose counter device shown in FIGs. 1 to 3. In FIG. 4a the dose counter device is shown at its position of rest and in FIG 4b it is shown at the position at the point of actuation of the dose counter device.
FIGURES 5 to 7 are side views of further exemplary dose counter devices in accordance with the invention.
FIGURE 8 is a partial cross-section of a yet further exemplary medicinal dispensing device (shown without a dispensing canister) in accordance with the invention.
FIGURE 9 is an isometric view of an additional exemplary medicinal dispensing device in accordance with the invention.
FIGURE 10 is a partial cross-section of an exemplary medicinal dispensing device provided with a collar in accordance with the invention.

While the above-identified figures set forth illustrative embodiments of the present invention, other embodiments are also contemplated, as noted in the disclosure. In all cases, this disclosure presents the invention by way of representation and not limitation.

### DETAILED DESCRIPTION OF ILLUSTRATIVE EMBODIMENTS

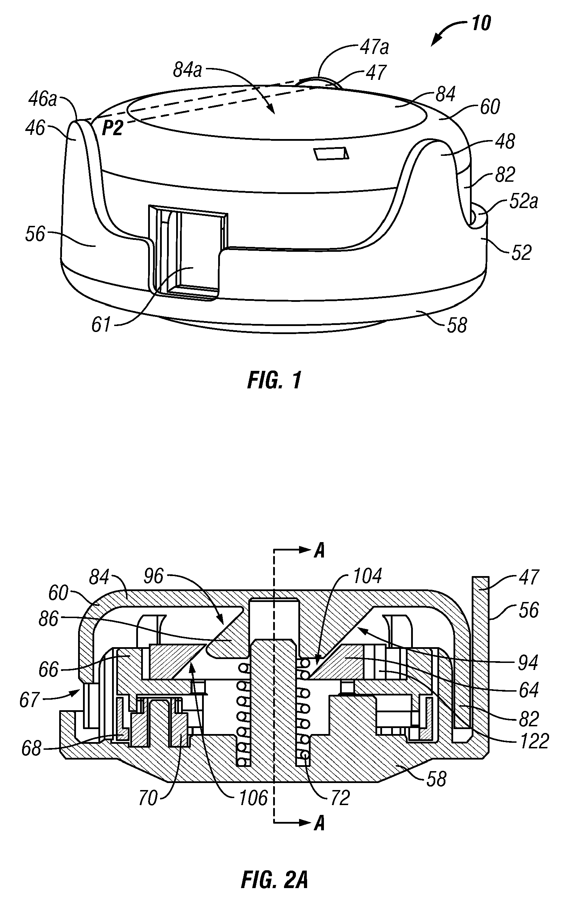

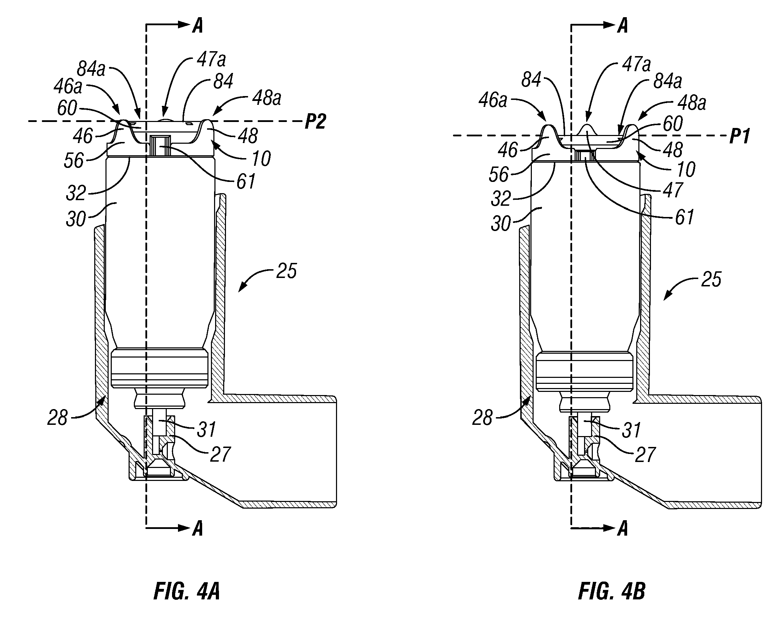

FIG. 1 shows an exemplary dose counter device (10) in accordance with the invention, while FIG 4 shows an exemplary medicinal dispensing device (25) (referred to as the dispenser in the following), in particular a metered dose inhaler, in accordance with the present invention including a dose counter device as shown in FIG. 1. The illustrated dose counter device (10) is shown at its rest position in FIGs. 1 and 4a and at the position at the point of actuation of the dose counter device in FIG. 4b. The dose counter device includes an actuation button (60) comprising a top plate (84) and a side wall (82). Referring to FIG. 4, the dispenser includes a dispensing canister (30) having an end equipped with a dispensing nozzle (31) and a closed end (32), and the dose counter device (10) is mounted point of actuation of the dose counter device in FIG. 4b. The dose counter device includes an actuation button (60) comprising a top plate (84) and a side wall (82). Referring to FIG. 4, the dispenser includes a dispensing canister (30) having an end equipped with a dispensing nozzle (31) and a closed end (32), and the dose counter device (10) is mounted to the closed end of the dispensing canister. To dispense a dose of medication and simultaneously actuate the dose counter device, the user applies a force in an axial direction A, by squeezing or pushing down on the actuation button, and thus the dispenser, in the axial direction causing a relative movement of the canister into a canister housing or adaptor (28) towards a support block (27) provided in the housing (compare FIG. 4a and 4b, e.g. the position of the dispensing canister within the housing and the position of the actuation button of the dose counter device).

To ensure that dose counter devices count each and every dose fired from such a medicinal dispensing device, typically such dose counter devices are designed to actuate under less force than the force necessary to actuate the dispenser and most often to actuate prior to the actuating of the dispenser. For this reason as mentioned above such counters are prone to unintentional actuation, e.g. inter alia when the dispenser rattles around or shifts about in a drawer or in a packaging carton or even during original assembly and/or transit within the manufacturer's supply chain of dose counter devices/dispensers.

It has been surprisingly found that by providing a parapet having two or more merlons as described herein that it is possible to minimize or essentially eliminate the potential for unintentional actuation of the dose counter device while still allowing the user good access to the counter actuation button to ensure proper and convenient operation during routine use of the dispenser. For example, the results of a series of 40 drop tests on metered dose inhalers (MDIs) including a top-mounted dose counter and parapets with two to four merlons as described herein showed a highly significant reduction (MDIs with two-merlon parapets) or essentially an elimination of unintentional actuation of the dose counter (MDIs wth three or four-merlon parapets) in comparison to such MDIs provided with no parapet and MDIs provided with a parapet including a single merlon.

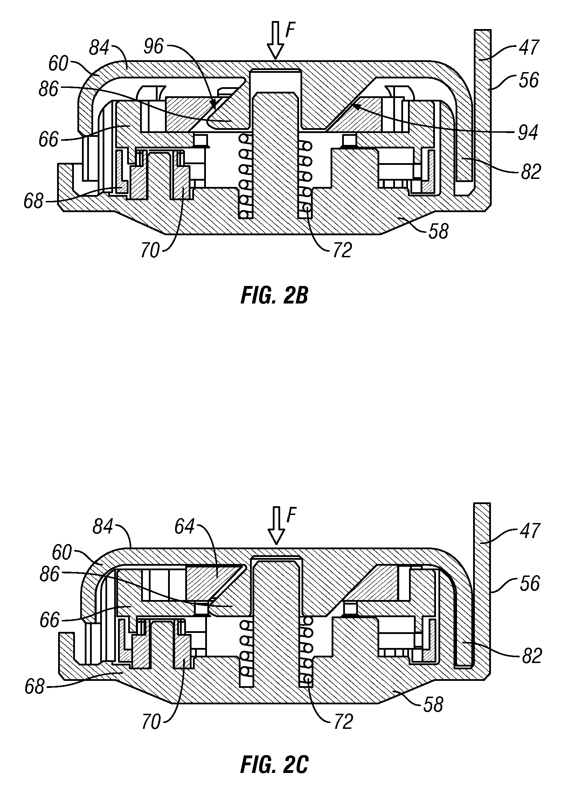

For example, returning to FIG. 4b which shows the dose counter device at the position at the point of actuation of the dose counter device, it can be seen that the dispenser, more particular the dose counter device, is provided with a parapet (56) with three merlons (46, 47 and 48). The most axially outwardly facing surfaces (46a, 47a, 48a) of the merlons extend outwardly beyond a first plane (P1) generally perpendicular to the axial direction and tangential to the most axially outwardly facing surface (84a) of the top plate (84) of the counter actuation button (60) at its position at the point of actuation of the dose counter device. Furthermore referring to FIG. 4a showing the dose counter device at its rest position, it can be seen that the most axially outwardly facing surfaces (46a, 47a and 48a) of the merlons desirably extend outwardly beyond a second plane (P2) generally perpendicular to the axial direction and tangential to the most axially outwardly facing surface (84a) of the top plate (84) of the counter actuation button (60) at the position of the button at rest. Referring to FIG. 1 showing an isometric view of just the dose counter device the outward extension of the three merlons (46, 47 and 48) can be better recognized, in particular by reference to a dashed line joining the surfaces (46a) to (47a) which is, in an axial direction, above the dashed line P2. Also it can be seen that the merlons are positioned around the counter actuation button (60) near its sidewall (82). This can be even better understood by viewing FIG. 2 showing the dose counter device of FIG. 1 and FIG. 4 in cross-section. FIG 2a is a cross section of the dose counter at rest, FIG. 2b at the position of the point of actuation (which could also be referred to as "the point of no return" i.e. where a release of force no longer prevents the dose counter device from registering a count) and FIG. 2c at the position of full depression of the counter actuation button. As can be seen in FIG. 2, the merlons (46, 47 and 48; only 47 is visible) are positioned near, but not in contact with the sidewall (82) of the actuation button (60). Also it can be recognized from FIG 2a to 2b that the parapet (56) with its merlons (46, 47 and 48) can be favorably provided as an integral part of a base (58) component of the dose counter device.

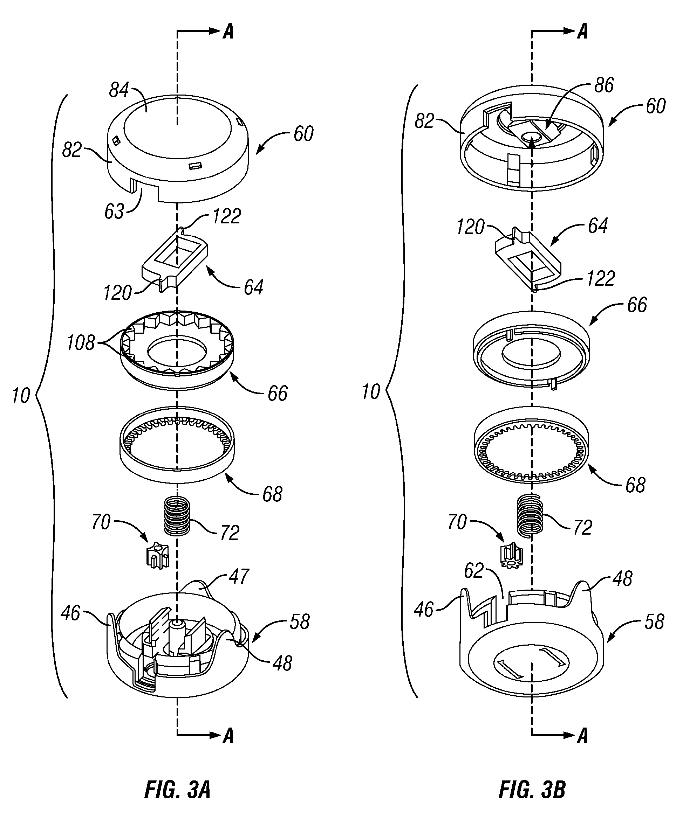

For the sake of completeness and understanding, the mechanism and operation of the dose counter device illustrated in FIGs. 1, 2 and 4 will be described in the following by making reference to FIG. 2 and to FIG. 3a and 3b showing exploded views of the components of said dose counter device. Referring to FIG. 2 it can be recognized that the counter actuation button (60) is coupled to the base (58) for axial, non-rotational movement with respect to the base, with the actuation button having a peg (86) thereon extending toward the base and where a compression spring (72) is disposed between the base and the actuation button to bias the actuation button away from the base. The dose counter device (10) also comprises a slider (64) non-rotatably disposed relative to the peg (86). The slider includes two fingers (120, 122) projecting from opposite ends thereof. This is best seen in FIG. 3. The slider (64) and the peg (86) having cooperating facing sliding surfaces (for example referring to FIG. 2a, the surfaces of the peg labeled 94 and 96 cooperate with the surface of the slider labeled 104 and 106, respectively). The illustrated dose counter device includes a first counter ring (66) rotatably disposed relative to the base. Referring to FIG. 3 and FIG. 2, it can be seen that the first counter ring has an inner surface comprising teeth (108) that are engageable by the fingers of the slider acting alternatively to cause indexed rotation of the first counter ring. The dose counter device is operated by an application of force (represented by the symbol F in FIGs. 2b and 2c) in an axial direction (in its intended use by the user) whereby the actuation button moves in a first direction (downward movement illustrated by comparison of FIG. 2a and 2b) along axis A. This movement of the actuation button in said first direction through the cooperating facing sliding surfaces of the peg and slider causes the slider (64) to move in a first linear direction laterally relative to axis A (movement to the right, illustrated by comparison of FIG. 2a to 2b to 2c). This movement of slider in the first lateral direction leads to engagement of the finger (122) of the slider with a tooth (108) of the first counter ring and then rotation of the first counter ring through a first arc in the first circumferential direction about axis A (in FIG. 3a, rotation in the clockwise direction). Then (not illustrated in FIG. 2) upon release of the actuation button (by the user in normal use) the actuation button moves in a second opposite direction (upwardly) due to the bias provided by the compression spring (72), which in turns moves the slider in a second opposite linear direction (to the left), which in turn allows engagement of the opposite slider finger (120) with a tooth of the first counter ring and thereby causes rotation of the first counter ring through a second arc in the first circumferential direction. The sum of the first and second arcs of movement of the first counter ring define a circumferential extent of movement of the first counter ring relative to the actuation button indicating a single count and, under its intentional use with a medicinal dispenser, e.g. a metered dose inhaler, that a single dose of aerosol medication was dispensed from the dispenser. From FIG. 2 and FIG. 3 it can be recognized that the dose counter includes a second counter ring (68), e.g. a "tens" counter ring, and a transfer gear (70) disposed between the first and second counter rings (66 and 68) and aligned to cause rotation of the second counter ring as a function of the rotation of the first counter ring. The indicia (61, not visible in FIG. 2) on the rings are viewable through cut-out portions (63,62) in the actuation button and the base, the cut-out portion forming a display window (67).

It will be appreciated by the skilled reader e.g. by viewing the exemplary dose counter device shown in FIG. 2, that the provision of the parapet with its merlons in the devices as described herein advantageously functions to guard against unintentional forces reaching the actuation button (e.g. the top plate or the shoulder between the top plate and side wall) as well as to reduce the magnitude of unintentional forces approaching the actuation button (e.g. in case an unintentional force manages to reach the actuation button).

The internal mechanism for counting in the dose counter device shown in FIGS. 1-4 is disclosed in our co-pending patent application no. PCT/US2007/063043. It will be understood that the provision of a parapet with two or more merlons as described here is equally applicable for other dose counter devices, e.g. end-mounted dose counter devices, that include other types of internal mechanisms for counting and in their proper and intended operation are operable by an application of a force by a user, and for other dispensers provided with such dose counter devices.

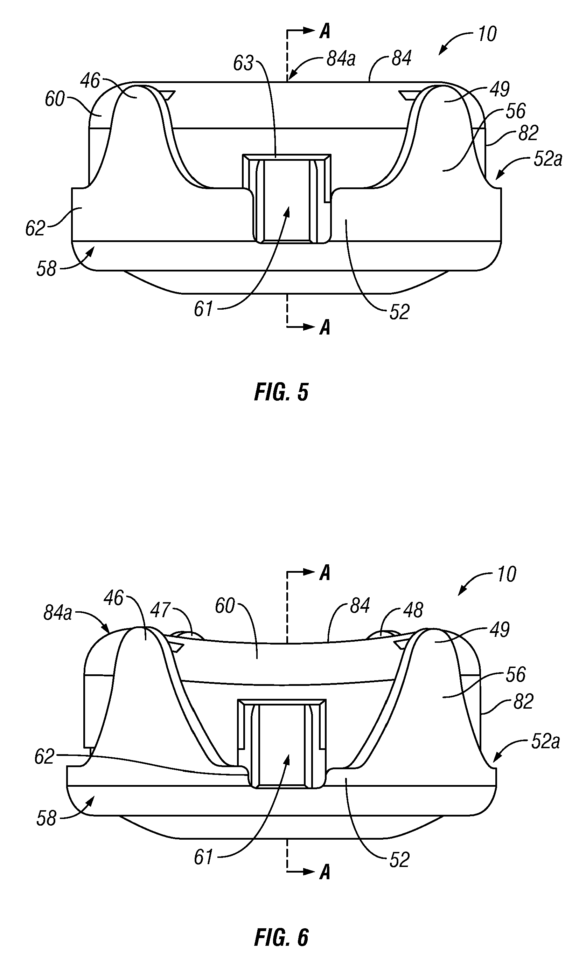

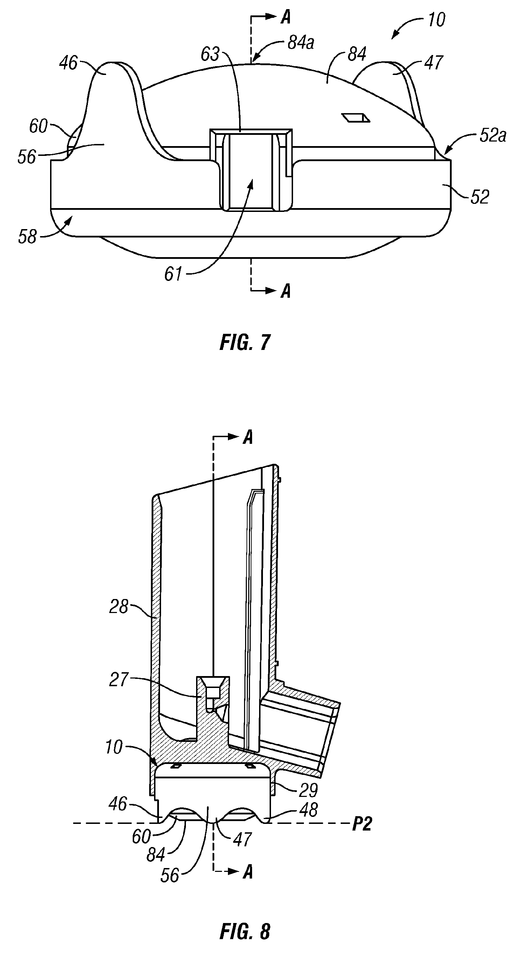

FIGURES 5 to 7 provide side views of further exemplary dose counter devices in accordance with the invention, each shown in their rest position. The dose counter device shown in FIG. 5 is similar to that shown in FIG. 1 in that the device (10) includes an actuation button with an essentially flat top plate (84). The illustrated dose counter device in FIG. 5 includes a parapet with four merlons (46 to 49; only 46 and 49 being visible) positioned around the actuation button near the sidewall (82). A plane tangential to the most axially outwardly facing surfaces of the merlons is slightly lower than the plane tangential to the most axially outwardly facing surface (84a) of the top plate of the actuation button in its rest position. FIG. 6 illustrates a dose counter device having an actuation button (60) with the top plate (84) being concave in form and a parapet including four merlons (46 to 49) positioned around the actuation button near the sidewall (82), where the most axially outwardly facing surfaces of the merlons are essentially flush with a plane generally perpendicular to the axial direction (A) and tangential to the most axially outwardly facing surfaces (84a) of the top plate of the actuation button in its rest position. FIG. 7 illustrates a dose counter device having an actuation button (60) with the top plate (84) being convex in form and a parapet including two merlons (46, 47) that are positioned essentially diametrically opposite each other around the actuation button near the sidewall (82), where the most axially outwardly facing surfaces of the merlons extend outwardly beyond a plane generally perpendicular to the axial direction (A) and tangential to the most axially outwardly facing surface (84a) of the top plate of the actuation button in its rest position. In each of the illustrated embodiments, the parapet (56) is provided as an extension from the base component (58) of the dose counter device (10). Referring to e.g. the illustrated embodiment shown in FIG. 7, the skilled reader will appreciate that counter actuation buttons can be designed such that the length of sidewall(s) of the actuation button is reduced down essentially to the thickness of the top plate.

FIG. 8 illustrates an alternative exemplary arrangement including a dose counter device (10) attached to an external end (29) of a housing (28) for a dispensing canister (not shown). Although the dispensing canister is not shown in FIG. 8, the skilled reader will recognized that the arrangement shown in FIG. 8 is for housing a dispensing canister equipped with a dispensing nozzle where the nozzle will be located in the support block (27) and the aforementioned external end (29) will be opposite to the closed end of the dispensing canister. The illustrated dose counter device (shown in its rest position) is hence a bottom-mounted dose counter device. Referring to FIG. 8 the dose counter device includes an actuation button (60) with an essentially flat top plate (84) and a parapet (56) with four merlons (46 to 49; only 46 to 48 are visible) equally spaced around the actuation button. It will be appreciated that the top plate (84) will, despite its name, be located in this embodiment at the bottom of the dose counter device in its normal orientation of use. The most axially outwardly facing surfaces of the merlons (46 to 49) extend outwardly beyond the plane (P2) that is generally perpendicular to the axial direction (A) and tangential to the most axially outwardly facing surface of the top plate (84) of the counter actuation button at its rest position. In alternative embodiments a parapet may be favorably mounted onto, or provided as an integral component of, the housing rather than as an integral component of the dose counter. In regard to the latter for example the merlons may be provided as extensions from the external end (29) of the housing.

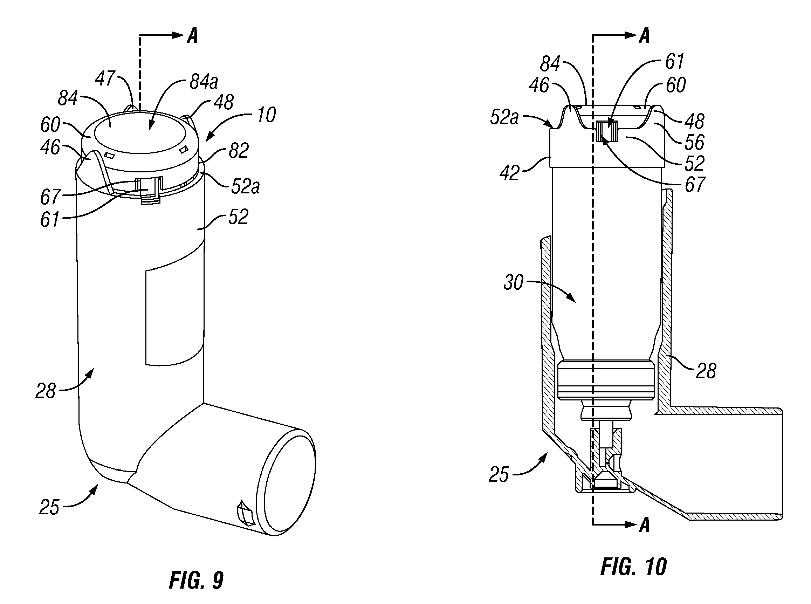

Also for top-mounted dose counter devices, a parapet may be mounted onto or provided as an integral component of the housing. For example in regard to the latter alternative this can be best understood by reference to FIG. 9 showing a medicinal dispensing device (25), in particular a metered dose inhaler, with a dose counter device (10) mounted to the closed end (not visible) of the dispensing canister (not visible). The illustrated dose counter device is shown in its rest position. As can be seen in FIG. 9, the dispensing canister housing (28) is provided with three, unequally-spaced, merlons (46, 47, 48) extending axially outwardly and positioned around the actuation button (60) near the sidewall (82) of the actuation button. The most axially outwardly facing surfaces of the merlons (46, 47, 48) extend outwardly beyond a plane generally perpendicular to the axial direction (A) and tangential to the most axially outwardly facing surface (84a) of the top plate (84) of the actuation button in its rest position.

FIGURES 1 to 9 illustrate exemplary embodiments in which the parapet is provided as an integral component of a medicinal dispensing device, e.g. an integral component of a dispensing-canister-housing, or as an integral component of a dose counter device, e.g. an integral component of the dose counter base component. Favorable embodiments also include embodiments in which a parapet is not integral with, but is mounted on, a component of a dose counter device, e.g. on a counter base or housing, or on a component of a medicinal dispensing device, e.g. on a dispensing canister or a dispensing canister housing. Such embodiments are favorable in that the parapet may be retrofitted. Moreover it has been found favorable to provide a parapet in the form of a collar, e.g. a closed or a split collar, that is adapted to be mounted on a component of a dose counter device or on a component of a medicinal dispensing device. FIG. 10 provides an illustration of an exemplary medicinal dispensing device, in particular a metered dose inhaler, comprising a dose counter device mounted on the closed end (not visible) of a dispensing canister (30), onto which a collar (42) provided with a parapet (56) including three merlons (46 to 48; only 46 and 48 are visible) has been mounted. In particular in the embodiment shown in FIG. 10 a lower cylindrical portion of the collar is mounted on the dispensing canister near its closed end, while an upper cylindrical portion of the collar including the parapet is positioned around the actuation button such that the merlons (46, 47, 48) are positioned around the counter actuation button near its sidewall (not visible) and the most axially outwardly facing surfaces of the merlons are flush with a plane generally perpendicular to axial direction (A) and tangential to the most axially outwardly facing surface of the top plate (84) of the counter actuation button at the position of the actuation button at rest.

Referring to FIGs. 1 to 10, the parapet may be provided with a wall or walls between merlons. In particular for example referring to FIG. 1 or FIGs. 5 to 7, it will be recognized that merlons can be provided as extensions out of a base component of a dose counter device without any wall or walls between the merlons. However it has been found that the provision of a wall or walls between merlons is favorable in e.g. providing additional structural stability and robustness to the parapet and merlons. Again referring FIG. 1 or FIGs. 5 to 7, between the merlons, walls (52) are provided. Some embodiments, such as the embodiment illustrated in FIG. 9 in which merlons are provided as extensions of a dispensing canister housing, will inherently have a wall or walls between merlons. Generally if there is such a wall or walls between merlons, desirably the most axially outwardly facing surface(s) (52a) of the wall(s) are flush with or, more favorably lower than, the plane generally perpendicular to the axial direction and tangential to the most axially outwardly facing surface(s) (84a) of the top plate (84) of the counter actuation button (60) at its position at the point of actuation of the dose counter device in order to facilitate the proper and intended use of the counter button and/or actuation of the dose counter device by the user. Also if needed or desired for ease in viewing of counting indicia (61) in a display window (67), a cut-out portion may be provided in such a wall.

The parapet includes two or more merlons. In embodiments including two merlons, desirably at least a portion of each merlon is diametrically opposite to the other. For example, in the exemplary embodiment shown in FIG. 7 it can be seen that the two merlons are positioned diametrically opposite, with equal spacing of the two gaps (crenales) between the two merlons. Embodiments including two merlons with unequally spacing of the two gaps between the two merlons can be envisioned. For example, the embodiment shown in FIG. 7 can be modified such that the width of each merlon is extended to the left relative to the drawing, so that the gap spanning across the display window is wider than the other gap and so that a portion of each merlon is diametrically opposite to the other. The use of three or more merlons is more favorable in minimizing or essentially preventing unintentional actuation of the dose counter devices. The upper boundary to the number of merlons is unlimited. However in regard to minimization of the potential for unintentional actuation as well as for ease in manufacturing and user-access to the counter actuation button typically 6 or less merlons is sufficient, more particular 5 or less, most particularly 4 or less. Again merlons may be equally or unequally distributed around the actuation button. For example, in the embodiment shown in FIG. 9 the three illustrated merlons (46, 47, 48) are unequally distributed, while in FIG. 1 the three illustrated merlons (46, 47, 48) are equally distributed. The positioning of the merlons is typically selected taking into consideration the number used, their purpose to minimize or essentially eliminate unintentional actuation of the dose counter device in light of the particular dose counter device (e.g. the position of the counting indicia display window), how it is mounted (e.g. top- or bottom-mounted) and ergonomic aspects (e.g. how it is intended that the user holds the dispenser and applies force to actuate the dispenser/dose counter device).

Desirably for easy access to the actuation counter button by the user, at half-height of the merlons, at least one gap (more favorably two gaps) between merlons has (have) a width near or greater than the width of an adult human forefinger, for example about 2 cm or more. Also for ease in accessing the actuation counter button for its intended use, it is favorable that at half-height of the merlons the width of at least one gap between merlons is equal to or greater than (more desirably greater than) the width of the merlons. More favorably at half-height of the merlons the width of two gaps between merlons is equal to or greater than (more desirably greater than) the width of the merlons, e.g. to allow easy access for both right-handed and left-handed users. For embodiments including three or more merlons, desirably at half-height of the merlons the width of all gaps between the merlons is equal to or greater than (more desirably greater than) the width of the merlons merlons have a substantially curved profile to provide stability and avoid inconvenience or injury for the user.

In all cases, this disclosure presents the invention by way of representation and not limitation. For example, it is favorable, as can be seen the exemplary embodiments shown in the FIGURES, that the merlons are positioned around the actuation counter button near an external surface of the sidewall of the actuation counter button. However embodiments can be envisioned in which merlons are positioned around actuation counter button near an internal surface of the sidewall of the actuation counter button, where the merlons extend outwardly through perforations provided in the actuation button, in particular through perforations in the top plate of the actuation button. Further, as can be seen the exemplary embodiments shown in the FIGURES, the merlons are desirably formed having the same shape and width, however, embodiments can be envisioned in which merlons are provided with differing widths and/or shapes.

## Claims

1. A medicinal dispensing device (25) comprising a dose counter device (10) for counting the doses of an aerosol medication dispensed from the dispensing device (25), said dispensing caused by an application of force in an axial direction on said dispensing device (25), the dose counter device (10) comprising an actuation button (60) and being operable by application of a force in the axial direction to the dispensing device (25), where the actuation button (60) comprises a top plate (84) and a side wall (82) or side walls, and wherein at the position of the counter actuation button (60) at the point of actuation of the dose counter device (10) there is a plane generally perpendicular to said axial direction and tangential to the most axially outwardly facing surface (84a) or surfaces of the top plate (84) of the counter actuation button (60) and wherein the dispensing device (25) is provided with a crenellated parapet (56) having two or more merlons (46; 47; 48; 49), the merlons (46; 47; 48; 49) being positioned around the counter actuation button (60), wherein the most axially outwardly facing surfaces (46a; 47a; 48a; 49a) of the merlons (46; 47; 48; 49) extend outwardly beyond said plane.

2. A device according to claim 1, wherein said plane is defined as a first plane and wherein at the position of the actuation button (60) at rest there is a second plane generally perpendicular to said axial direction and tangential to the most axially outwardly facing surface (84a) or surfaces of the top plate (84) of the counter actuation button (60) and wherein the most axially outwardly facing surfaces (46a; 47a; 48a; 49a) of the merlons (46; 47; 48; 49) are flush with or extend outwardly beyond the second plane.

3. A device according to claim 1 or 2, wherein the parapet (56) is mounted on a component of the dispensing device (25) or integral with a component of the dispensing device, said component not being the counter actuation button (60) of the dose counter device (10).

4. A device according to any one of claims 1 to 3, wherein the dispensing device (25) comprises a dispensing canister (30) having an end equipped with a dispensing nozzle (31) and a closed end (32), the dose counter device (10) being mounted to the closed end (32) of the dispensing canister (30).

5. A device according to any one of claims 1 to 3, wherein the dispensing device (25) comprises a dispensing canister (30) having an end equipped with a dispensing nozzle (31) and a closed end (32) and a housing for the dispensing canister (30), the dose counter device (10) being mounted on, or being integral with, an external end of the housing that is opposite to the closed end (32) of the canister (32).

6. A dose counter device (10) for use on a medicinal dispensing device (25) and for counting the doses of an aerosol medication dispensed from a medicinal dispensing device (25), the dose counter device (10) comprising an actuation button (60) and being operable by application of a force in an axial direction on said actuation button (60), where the actuation button (60) comprises a top plate (84) and a side wall (82) or side walls, and
wherein at the position of the actuation button (60) at the point of actuation of the dose counter device (10) there is a plane generally perpendicular to said axial direction and tangential to the most axially outwardly facing surface (84a) or surfaces of the top plate (84) of the counter actuation button (60) and wherein the dose counter device (10) is provided with a crenellated parapet (56) having two or more merlons (46; 47; 48; 49), the merlons (46; 47; 48; 49) being positioned around the counter actuation button (60), wherein the most axially outwardly facing surfaces (46a; 47a; 48a; 49a) of the merlons (46; 47; 48; 49) extend outwardly beyond said plane.

7. A device according to claim 6, wherein said plane is defined as a first plane and wherein at the position of the actuation button (60) at rest there is a second plane generally perpendicular to said axial direction and tangential to the most axially outwardly facing surface (84a) or surfaces of the top plate (84) of the counter actuation button (60) and wherein the most axially outwardly facing surfaces (46a; 47a; 48a; 49a) of the merlons (46; 47; 48; 49) are flush with or extend outwardly beyond the second plane.

8. A device according to claim 6 or claim 7, where said dispensing device (25) comprises a dispensing canister (30) having an end equipped with a dispensing nozzle (31) and a closed end (32), and wherein the dose counter device (10) is adapted to be mounted to the closed end (32) of the dispensing canister (30); or where said dispensing device (25) comprises a dispensing canister (30) having an end equipped with a dispensing nozzle (31) and a closed end (32) and a housing for the dispensing canister (30), and wherein the dose counter device (10) is adapted to be mounted to an external end of the housing that is opposite to the closed end (32) of the canister (30).

9. A device according to any one of claims 6 to 8, wherein the merlons (46; 47; 48; 49) are positioned around the actuation button (60) near an internal surface of the sidewall (82) of the counter actuation button (60), where the merlons (46; 47; 48; 49) extend outwardly through perforations provided in the counter actuation button (60).

10. A device according to any one of claims 1 to 8, wherein the merlons (46; 47; 48; 49) are positioned around the counter actuation button (60) near an external surface of the sidewall (82) of the counter actuation button (60).

11. A device (25; 10) according to any one of claims 1 to 10, wherein at half-height of the merlons (46; 47; 48; 49), the width of at least one gap between merlons (46; 47; 48; 49) is equal to or greater than about
2 cm; and/or at half-height of the merlons (46; 47; 48; 49), the width of at least one gap between merlons (46; 47; 48; 49) is equal to or greater than the width of the merlons (46; 47; 48; 49) at half-height.

12. A device (25; 10) according to any one of claims 1 to 11, wherein the parapet (56) includes three or more
merlons (46; 47; 48; 49).

## Patentansprüche

1. Medikamentenabgabevorrichtung (25), die eine Dosiszählervorrichtung (10) zum Zählen der Dosen eines von einer Abgabevorrichtung (25) abgegebenen Medikamentenaerosols umfasst, wobei die Abgabe durch in Axialrichtung auf die Abgabevorrichtung (25) angewendete Kraft verursacht wird, wobei die Dosiszählervorrichtung (10) einen Auslöseknopf (60), der durch Anwenden einer Kraft auf die Abgabevorrichtung (25) in Axialrichtung betätigt wird, umfasst, wobei der Auslöseknopf (60) eine Kopfplatte (84) und eine Seitenwand (82) oder Seitenwände umfasst, und wobei sich an der Stelle des Zählerauslöseknopfes (60) zum Zeitpunkt der Betätigung der Dosiszählervorrichtung (10) eine Ebene befindet, die allgemein perpendikulär zur Axialrichtung und tangential zur am stärksten nach außen gerichteten Oberfläche (84a) oder den Oberflächen der Kopfplatte (84) des Zählerauslöseknopfes (60) verläuft, und wobei die Abgabevorrichtung (25) mit einem zinnenartigen Wall (56) mit zwei oder mehreren Zinnen (46; 47; 48; 49) ausgestattet ist, wobei die Zinnen (46; 47; 48; 49) um den Zählerauslöseknopf (60) herum angeordnet sind, wobei sich die am stärksten nach außen gerichteten Flächen (46a; 47a; 48a; 49a) der Zinnen (46; 47; 48; 49) nach außen über die Ebene hinweg erstrecken.

2. Vorrichtung nach Anspruch 1, wobei die Ebene als eine erste Ebene definiert ist, und wobei sich an der Stelle des Auslöseknopfes (60) in Ruhestellung eine zweite Ebene befindet, die allgemein perpendikulär zur Axialrichtung und tangential zu der Oberfläche (84a) oder den Oberflächen an der Kopfplatte (84) des Zählerauslöseknopfes (60), die am meisten nach außen gerichtet sind, verläuft, und wobei die am stärksten nach außen gerichteten Flächen (46a; 47a; 48a; 49a) der Zinnen (46; 47; 48; 49) mit der zweiten Ebene bündig sind oder sich über diese hinaus erstrecken.

3. Vorrichtung nach Anspruch 1 oder 2, wobei der Wall (56) auf einem Bestandteil der Abgabevorrichtung (25) montiert ist oder in einen Bestandteil der Abgabevorrichtung eingebaut ist, wobei der Bestandteil nicht der Zählerauslöseknopf (60) der Dosiszählervorrichtung (10) ist.

4. Vorrichtung nach einem der Ansprüche 1 bis 3, wobei die Abgabevorrichtung (25) einen Abgabebehälter (30) mit einem mit einer Abgabedüse (31) ausgestatteten Ende und einem geschlossenen Ende (32) aufweist, wobei die Dosiszählervorrichtung (10) an dem geschlossenen Ende (32) des Abgabebehälters (30) montiert ist.

5. Vorrichtung nach einem der Ansprüche 1 bis 3, wobei die Abgabevorrichtung (25) einen Abgabebehälter (30) mit einem mit einer Abgabedüse (31) ausgestatteten Ende und einem geschlossenen Ende (32) und ein Gehäuse für den Abgabebehälter (30) aufweist, wobei die Dosiszählervorrichtung (10) an einem dem geschlossenen Ende (32) des Behälters (32) entgegengesetzten, externen Ende des Gehäuses montiert oder darin eingebaut ist.

6. Dosiszählervorrichtung (10) für die Verwendung an einer Medikamentenabgabevorrichtung (25) und zum Zählen der Dosen eines von einer Abgabevorrichtung (25) abgegebenen Medikamentenaerosols, wobei die Dosiszählervorrichtung (10) einen Auslöseknopf (60) umfasst und durch Anwenden einer Kraft auf den Auslöseknopf (60) in Axialrichtung betätigt wird, wobei der Auslöseknopf (60) eine Kopfplatte (84) und eine Seitenwand (82) oder Seitenwände umfasst, und
wobei sich an der Stelle des Zählerauslöseknopfes (60) zum Zeitpunkt der Betätigung der Dosiszählervorrichtung (10) eine Ebene befindet, die allgemein perpendikulär zur Axialrichtung und tangential zu der Oberfläche (84a) oder den Oberflächen an der Kopfplatte (84) des Zählerauslöseknopfes (60), die am stärksten nach außen gerichtet sind, verläuft, und wobei die Dosiszählervorrichtung (10) mit einem zinnenartigen Wall (56) mit zwei oder mehreren Zinnen (46; 47; 48; 49) ausgestattet ist, wobei die Zinnen (46; 47; 48; 49) um den Zählerauslöseknopf (60) herum angeordnet sind, wobei sich die am stärksten nach außen gerichteten Flächen (46a; 47a; 48a; 49a) der Zinnen (46; 47; 48; 49) nach außen über die Ebene hinweg erstrecken.

7. Vorrichtung nach Anspruch 6, wobei die Ebene als eine erste Ebene definiert ist, und wobei sich an der Stelle des Auslöseknopfes (60) in Ruhestellung eine zweite Ebene befindet, die allgemein perpendikulär zur Axialrichtung und tangential zu der Oberfläche (84a) oder den Oberflächen an der Kopfplatte (84) des Zählerauslöseknopfes (60), die am meisten nach außen gerichtet sind, verläuft, und wobei die am stärksten nach außen gerichteten Flächen (46a; 47a; 48a; 49a) der Zinnen (46; 47; 48; 49) mit der zweiten Ebene bündig sind oder sich über diese hinaus erstrecken.

8. Vorrichtung nach Anspruch 6 oder Anspruch 7, wobei die Abgabevorrichtung (25) einen Abgabebehälter (30) mit einem mit einer Abgabedüse (31) ausgestatteten Ende und einem geschlossenen Ende (32) aufweist, und wobei die Dosiszählervorrichtung (10) konzipiert ist, um an dem geschlossenen Ende (32) des Abgabebehälters (30) montiert zu werden; oder wobei die Abgabevorrichtung (25) einen Abgabebehälter (30) umfasst, der ein mit einer Abgabedüse (31) ausgestattetes Ende und ein geschlossenes Ende (32) und ein Gehäuse für den Abgabebehälter (30) aufweist, und wobei die Dosiszählervorrichtung (10) konzipiert ist, um an einem dem geschlossenen Ende (32) des Abgabebehälters (30) entgegengesetzten, externen Ende des Gehäuses montiert zu werden.

9. Vorrichtung nach einem der Ansprüche 6 bis 8, wobei die Zinnen (46; 47; 48; 49) um den Auslöseknopf (60) herum nahe einer Innenoberfläche der Seitenwand (82) des Auslöseknopfes (60) angeordnet sind, wobei sich die Zinnen (46; 47; 48; 49) durch in dem Zählerauslöseknopf (60) bereitgestellten Perforationen nach außen erstrecken.

10. Vorrichtung nach einem der Ansprüche 1 bis 8, wobei die Zinnen (46; 47; 48; 49) um den Zählerauslöseknopf (60) herum nahe einer Außenoberfläche der Seitenwand (82) des Auslöseknopfes (60) angeordnet sind.

11. Vorrichtung (25; 10) nach einem der Ansprüche 1 bis 10, wobei auf halber Höhe der Zinnen (46; 47; 48; 49) die Breite von mindestens einem Zwischenraum zwischen den Zinnen (46; 47; 48; 49) gleich oder größer als etwa 2 cm ist; und/oder auf halber Höhe der Zinnen (46; 47; 48; 49) die Breite von mindestens einem Zwischenraum zwischen den Zinnen (46; 47; 48; 49) gleich der Breite oder größer als die Breite der Zinnen (46; 47; 48; 49) auf halber Höhe ist.

12. Vorrichtung (25; 10) nach einem der Ansprüche 1 bis 11, wobei der Wall (56) drei oder mehr Zinnen (46; 47; 48; 49) beinhaltet.

## Revendications

1. Dispositif distributeur médicinal (25) comprenant un dispositif compteur de dose (10) destiné à compter les doses d'une médication en aérosol distribuée à partir d'un dispositif distributeur (25), ladite distribution étant provoquée par l'application d'une force dans une direction axiale sur ledit dispositif distributeur (25), le dispositif compteur de dose (10) comprenant un bouton d'actionnement (60) et étant opérationnel par l'application d'une force dans la direction axiale au dispositif distributeur (25), où le bouton d'actionnement (60) comprend une plaque supérieure (84) et une paroi latérale (82) ou des parois latérales, et dans lequel, à la position du bouton d'actionnement de compteur (60) au niveau du point d'actionnement du dispositif compteur de dose (10), il y a un plan généralement perpendiculaire à ladite direction axiale et tangentiel à la surface (84a) ou aux surfaces les plus axialement tournées vers l'extérieur de la plaque supérieure (84) du bouton d'actionnement de compteur (60) et où le dispositif distributeur (25) est pourvu d'un parapet crénelé (56) comportant deux merlons ou plus (46 ; 47 ; 48 ; 49), les merlons (46 ; 47 ; 48 ; 49) étant positionnés autour du bouton d'actionnement de compteur (60), dans lequel les surfaces les plus axialement tournées vers l'extérieur (46a ; 47a ; 48a ; 49a) des merlons (46 ; 47 ; 48 ; 49) s'étendent vers l'extérieur au-delà dudit plan.

2. Dispositif selon la revendication 1, dans lequel ledit plan est défini en tant que premier plan et dans lequel, à la position du bouton d'actionnement (60) au repos, il y a un deuxième plan généralement perpendiculaire à ladite direction axiale et tangentiel à la surface (84a) ou aux surfaces les plus axialement tournées vers l'extérieur de la plaque supérieure (84) du bouton d'actionnement de compteur (60) et dans lequel les surfaces les plus axialement tournées vers l'extérieur (46a ; 47a ; 48a ; 49a) des merlons (46 ; 47 ; 48 ; 49) sont affleurantes au, ou s'étendent vers l'extérieur au-delà du, deuxième plan.

3. Dispositif selon la revendication 1 ou 2, dans lequel le parapet (56) est monté sur un composant du dispositif distributeur (25) ou est solidaire d'un composant du dispositif distributeur, ledit composant n'étant pas le bouton d'actionnement de compteur (60) du dispositif compteur de dose (10).

4. Dispositif selon l'une quelconque des revendications 1 à 3, dans lequel le dispositif distributeur (25) comprend un récipient distributeur (30) possédant une extrémité équipée d'un embout de distribution (31) et une extrémité fermée (32), le dispositif compteur de dose (10) étant monté sur l'extrémité fermée (32) du récipient distributeur (30).

5. Dispositif selon l'une quelconque des revendications 1 à 3, dans lequel le dispositif distributeur (25) comprend un récipient distributeur (30) possédant une extrémité équipée d'un embout de distribution (31) et une extrémité fermée (32) et un logement pour le récipient distributeur (30), le dispositif compteur de dose (10) étant monté sur, ou étant solidaire de, une extrémité externe du logement qui est opposée à l'extrémité fermée (32) du récipient (32).

6. Dispositif compteur de dose (10) pour une utilisation sur un dispositif distributeur médicinal (25) et destiné au comptage des doses d'une médication en aérosol distribuée à partir d'un dispositif distributeur médicinal (25), le dispositif compteur de dose (10) comprenant un bouton d'actionnement (60) et étant opérationnel par l'application d'une force dans une direction axiale sur ledit bouton d'actionnement (60), où le bouton d'actionnement (60) comprend une plaque supérieure (84) et une paroi latérale (82) ou des parois latérales, et
dans lequel, à la position du bouton d'actionnement (60) au niveau du point d'actionnement du dispositif compteur de dose (10), il y a un plan généralement perpendiculaire à ladite direction axiale et tangentiel à la surface (84a) ou aux surfaces les plus axialement tournées vers l'extérieur de la plaque supérieure (84) du bouton d'actionnement de compteur (60) et dans lequel le dispositif compteur de dose (10) est pourvu d'un parapet crénelé (56) possédant deux merlons ou plus (46 ; 47 ; 48 ; 49), les merlons (46 ; 47 ; 48 ; 49) étant positionnés autour du bouton d'actionnement de compteur (60), dans lequel les surfaces les plus axialement tournées vers l'extérieur (46a ; 47a ; 48a ; 49a) des merlons (46 ; 47 ; 48 ; 49) s'étendent vers l'extérieur au-delà dudit plan.

7. Dispositif selon la revendication 6, dans lequel ledit plan est défini en tant que premier plan et dans lequel, à la position du bouton d'actionnement (60) au repos, il y a un deuxième plan généralement perpendiculaire à ladite direction axiale et tangentiel à la surface (84a) ou aux surfaces les plus axialement tournées vers l'extérieur de la plaque supérieure (84) du bouton d'actionnement de compteur (60) et dans lequel les surfaces les plus axialement tournées vers l'extérieur (46a ; 47a ; 48a ; 49a) des merlons (46 ; 47 ; 48 ; 49) sont affleurantes au, ou s'étendent vers l'extérieur au-delà du, deuxième plan.

8. Dispositif selon la revendication 6 ou la revendication 7, où ledit dispositif distributeur (25) comprend un récipient distributeur (30) possédant une extrémité équipée d'un embout de distribution (31) et une extrémité fermée (32), et dans lequel le dispositif compteur de dose (10) est conçu pour être monté sur l'extrémité fermée (32) du récipient distributeur (30) ; ou dans lequel ledit dispositif distributeur (25) comprend un récipient distributeur (30) possédant une extrémité équipée d'un embout de distribution (31) et une extrémité fermée (32) et un logement pour le récipient distributeur (30), et dans lequel le dispositif compteur de dose (10) est conçu pour être monté sur une extrémité externe du logement qui est opposée à l'extrémité fermée (32) du récipient (30).

9. Dispositif selon l'une quelconque des revendications 6 à 8, dans lequel les merlons (46 ; 47 ; 48 ; 49) sont positionnés autour du bouton d'actionnement (60) près d'une surface interne de la paroi latérale (82) du bouton d'actionnement de compteur (60), où les merlons (46 ; 47 ; 48 ; 49) s'étendent vers l'extérieur à travers des perforations ménagées dans le bouton d'actionnement de compteur (60).

10. Dispositif selon l'une quelconque des revendications 1 à 8, dans lequel les merlons (46 ; 47 ; 48 ; 49) sont positionnés autour du bouton d'actionnement de compteur (60) près d'une surface externe de la paroi latérale (82) du bouton d'actionnement de compteur (60).

11. Dispositif (25 ; 10) selon l'une quelconque des revendications 1 à 10, dans lequel, à mi-hauteur des merlons (46 ; 47 ; 48 ; 49), la largeur d'au moins un écartement entre les merlons (46 ; 47 ; 48 ; 49) est égale ou supérieure à environ 2 cm ; et/ou, à mi-hauteur des merlons (46 ; 47 ; 48 ; 49), la largeur d'au moins un écartement entre les merlons (46 ; 47 ; 48 ; 49) est égale ou supérieure à la largeur des merlons (46 ; 47 ; 48 ; 49) à mi-hauteur.

12. Dispositif (25 ; 10) selon l'une quelconque des revendications 1 à 11, dans lequel le parapet (56) inclut trois merlons ou plus (46 ; 47 ; 48 ; 49).
